(19) Europäisches Patentamt
European Patent Office
Office européen des brevets

(11) EP 3 521 275 B1

(12) **EUROPEAN PATENT SPECIFICATION**

(45) Date of publication and mention of the grant of the patent:
**03.12.2025 Bulletin 2025/49**

(21) Application number: **17854878.0**

(22) Date of filing: **27.09.2017**

(51) International Patent Classification (IPC):
***C07D 209/86*** *(2006.01)* ***A61K 31/403*** *(2006.01)*
***A61P 3/00*** *(2006.01)* ***A61P 3/10*** *(2006.01)*

(52) Cooperative Patent Classification (CPC):
**A61K 31/403; A61P 3/00; A61P 3/04; A61P 3/10; A61P 9/00; A61P 9/10; A61P 25/00; C07D 209/86**

(86) International application number:
**PCT/CN2017/103619**

(87) International publication number:
**WO 2018/059428 (05.04.2018 Gazette 2018/14)**

(54) **SUBSTITUTED PHENYLPROPIONIC ACID ENANTIOMER AND MANUFACTURING METHOD, COMPOSITION, AND APPLICATION OF SAME**

SUBSTITUIERTES PHENYLPROPIONSÄURE-ENANTIOMER UND HERSTELLUNGSVERFAHREN, ZUSAMMENSETZUNG UND ANWENDUNG DAVON

ÉNANTIOMÈRE D'ACIDE PHÉNYLPROPIONIQUE SUBSTITUÉ, PROCÉDÉ DE FABRICATION, COMPOSITION ET APPLICATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2016 CN 201610856914**

(43) Date of publication of application:
**07.08.2019 Bulletin 2019/32**

(73) Proprietor: **Shenzhen Chipscreen Biosciences Co., Ltd.**
**Shenzhen, Guangdong 518057 (CN)**

(72) Inventors:
- **YU, Jindi**
  **Shenzhen, Guangdong 518057 (CN)**
- **PAN, Desi**
  **Shenzhen, Guangdong 518057 (CN)**
- **SHAN, Song**
  **Shenzhen, Guangdong 518057 (CN)**
- **LI, Zhibin**
  **Shenzhen, Guangdong 518057 (CN)**
- **LU, Xianping**
  **Shenzhen, Guangdong 518057 (CN)**

(74) Representative: **Lavoix**
**Bayerstraße 83**
**80335 München (DE)**

(56) References cited:
**WO-A1-2004/048333 CN-A- 1 562 970**
**CN-A- 105 801 468**

- **PING-PING LI ET AL: "The PPAR [alpha] / [gamma] dual agonist chiglitazar improves insulin resistance and dyslipidemia in MSG obese rats", BRITISH JOURNAL OF PHARMACOLOGY, vol. 148, no. 5, 1 July 2006 (2006-07-01), UK, pages 610 - 618, XP055669192, ISSN: 0007-1188, DOI: 10.1038/sj.bjp.0706745**
- **BHUSHAN RAVI ET AL: "Resolution of Enantiomers of Ibuprofen by Liquid Chromatography: A Review", BIOMEDICAL CHROMATOGRAPHY, JOHN WILEY & SONS LTD, GB, vol. 12, no. 6, 1 November 1998 (1998-11-01), pages 309 - 316, XP002668423, ISSN: 0269-3879, DOI: 10.1002/(SICI)1099-0801(199811/12) 12:6<309::AID-BMC763>3.0.CO;2-K**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **G�BITZ G ED - FELINGER ATTILA: "Separation of Drug Enantiomers by HPLC Using Chiral Stationary Phases A Selective Review", CHROMATOGRAPHIA, VIEWEG UND TEUBNER VERLAG, DE, vol. 30, no. 9-10, 1 November 1990 (1990-11-01), pages 555 - 564, XP002668422, ISSN: 0009-5893, DOI: 10.1007/BF02269804**
- **ASHOK K PEEPLIWAL ET AL: "A Review: Stereochemical consideration and eudismic ratio in chiral drug development", JOURNAL OF BIOMEDICAL SCIENCES AND RESEARCH, PHARMA INFO PUBLICATIONS, IN, vol. 2, no. 1, 1 January 2010 (2010-01-01), pages 29 - 45, XP007915083, ISSN: 0975-542X**
- **LAN, YUKUN ET AL.: "Synthesis of Novel Insulin Sensitizer Sigolide", CHINESE JOURNAL OF NEW DRUGS, vol. 13, no. 8, 31 August 2004 (2004-08-31), pages 718 - 720, XP009513907, ISSN: 1003-3734**
- **YAN, LIANGPING ET AL.: "In Vitro Metabolism of Bevacizat Sodium", CHINESE JOURNAL OF PHARMACOLOGY AND TOXICOLOGY, vol. 21, no. 2, 30 April 2007 (2007-04-30), pages 124 - 130, XP009513911, ISSN: 1000-3002**
- **LAN, YUKUN ET AL.: "M. Sc. Thesis", SYNTHESIS AND ANALYSIS OF SEVERAL TYROSINE DERIVATIVES WITH ANTI-TYPE II DIABETES, vol. B016, no. 2005/01, 15 March 2005 (2005-03-15), pages 1 - 57, XP009515757**


Remarks:

The file contains technical information submitted after the application was filed and not included in this specification

## Description

### CROSS REFERENCE TO RELATED APPLICATIONS

This application claims the priority of Chinese Patent Application No. 201610856914.7, filed on September 27, 2016, and titled with "(-)-2-[(2-(4-FLUOROBENZOYL)PHENYL)AMINO]-3-[(4-(2-CARBAZOLE-ETHOXY)PHE NYL)] PROPIONIC ACID, MANUFACTURING METHOD, COMPOSITION AND APPLICATION OF SAME".

### FIELD

The present invention relates to the field of pharmaceutical technology, specifically to the (-)-enantiomer of 2-[(2-(4-fluorobenzoyl)phenyl)amino]-3-[(4-(2-carbazole-ethoxy) phenyl)]propionic acid and pharmaceutically acceptable salt thereof, preparation method thereof, composition containing the same, and application thereof.

### BACKGROUND

PPARγ is a member of nuclear receptor superfamily that is primarily expressed in adipose tissue. PPARα, another member of this family, is expressed primarily in liver tissue and can be activated by a class of ligands called fibrates and reduce the levels of triglyceride and cholesterol. PPARα, can stimulate the proliferation of peroxidase and accelerates the oxidation of fatty acids, thereby reducing the fatty acid content in the blood (Keller and Wahli: Trends Endocrin Metab 1993, 4:291-296). It has recently been reported that PPARδ acts as a regulator of lipid metabolism with extensive fat-"burning" effects. In *in vitro* experiments, activation of PPARδ in adipose and skeletal muscle cells contributes to the oxidation and utilization of fatty acids. In animal adipose tissue with low PPARα, expression, activation of PPARδ can specifically induce expression of genes involved in fatty acid oxidation and energy expenditure, thereby improving lipid content and reducing weight. More importantly, these animals with activated PPARδ were completely resistant to high-fat diets as well as genetic factors (Lepr (db/db))-induced obesity. Short-term treatment of Lepr (db/db) mice with PPARδ activator can consume accumulated fat, whereas treating PPARδ-deficient mice with a high-fat diet can induce obesity (Wang YX et al., Cell 2003 Apr, 18; 113 (2): 159-70).

PPARα, PPARγ, and PPARδ can each form heterodimers with the retinoic acid X receptor. Therefore, RXR/PPAR heterodimers also play an important role in regulating the balance of cellular sugar and lipid and adipocyte differentiation. It has been reported that some novel compounds including activators of PPARγ and dual activators of PPARα/PPARγ have a good effect in preventing and treating metabolic syndrome in humans and animals (WO 00/08002, WO01/57001 A1, US6054453, EP088317 B1, WO97/25042, WO02/26729 A2 and US6353018 B1). Therefore, screening for new compounds with PPARα, PPARγ and PPARδ activator properties is of great importance for the comprehensive treatment of metabolic disorders. These metabolic disorders include metabolic syndromes such as diabetes, hypertension, obesity, insulin resistance, hypertriglyceridemia, hyperglycemia, high cholesterol, arteriosclerosis, coronary heart disease, and other cardiovascular diseases.

The applicant discloses a class of aralkyl amino acid activators of PPARα,γ and δ in Chinese patent CN1257893C, that have excellent hypoglycemic and lipid-lowering activity, wherein example 15 specifically discloses 2-[(2-(4-fluorobenzoyl)phenyl)amino]-3-[(4-(2-carbazole-ethoxy)phenyl)]propionic acid and the preparation method thereof and examples 21-28 disclose the pharmacological activity of the compound. 2-[(2-(4-fluorobenzoyl)phenyl) amino]-3-[(4-(2-carbazole-ethoxy)phenyl)]propionic acid is a racemate, whereas Chinese patent CN1257893 does not disclose its (+) or (-) enantiomer, nor the preparation method and pharmacological activity thereof. The synthesis of (2S)-3-[4-(-carbazol-9-ylethoxy)phenyl]-2-[2-(4-fluorobenzoyl)aniline]propanoic acid or chiglitazar corresponding to the (+) form or the dextroform has been disclosed (Lan Y-K et al., Chinese J New Drugs, 2004, 13(8): 718-720).

Generally, a compound will have multiple pairs of enantiomers that are mirror images of each other based on the difference in chiral position. Except for optical activity, the two enantiomers mirrored to each other generally have the same physical properties. In particular, the two enantiomers are generally completely or nearly identical in melting point, boiling point, solubility, density and refractive rate, but are completely opposite in terms of optical rotation. Since the two enantiomers rotated the plane of polarized light to the same extent, but in opposite directions, no net optical rotation will be observed when they are mixed. In other words, the optical rotation of the racemate is theoretically zero, and practically close to zero. Due to differences in optical activity, racemates and individual enantiomers may differ in their physiological activity and toxicity. However, this difference in physiological activity and toxicity is usually unpredictable, so it is not possible to predict from the difference in optical rotation in advance. In general, in most cases, the physiological activities (including pharmacodynamic effects) of the two isomers and the physicochemical properties other than the optical activity are identical. In rare cases, there exit certain pharmacodynamic differences. From the point of view of the difference in pharmacodynamics, there are mainly the following types of conditions. 1) The enantiomers have similar pharmaceutical property, but the activity intensity is different. For example, the antibacterial activity of ofloxacin S-isomer is 8-135 times

stronger than the R-isomer (Mitscher LA et al., J Med Chem, 1987, 30(12): 2283-6). 2) The enantiomers are opposite in pharmaceutical property and mutually antagonistic. For example, the (+)-isomer of Picenadol is an opioid receptor agonist and the (-)-isomer is an opioid receptor antagonist (Carter RB et al., J Pharmacol Exp Ther, 1985, 234 (2): 299-306). 3) The enantiomers are complementary in pharmaceutical property to each other. For example, the R isomer of the diuretic Indacrinone has a diuretic effect, as well as a side effect of increasing uric acid in the blood, while the S isomer promotes the excretion of uric acid and can effectively reduce the side effect of the R isomer. Therefore, it is beneficial to use them in combination. Further studies have shown that the therapeutic effect is the best when the ratio of S to R isomer is 1:4 or 1:8 (Tobert J. A. et al., Clin Pharmacol Ther, 1981, 29(3): 344-50). 4) Different enantiomers have different pharmacological activities. For example, the antihypertensive drug labetalol has two chiral centers, wherein the (R,R)-isomer is a non-selective β-adrenoceptor antagonist, the (S,R)-isomer is an α1-adrenoceptor antagonist, the (S,S)-isomer has weak α1-adrenoceptor blockade, while the (R,S)-isomer is inactive (Pieter A. et al., Drugs, 1993, 45(4): 509-517). 5) One of the enantiomers has strong toxic side effects. For example, thalidomide has been used as a sedative to treat pregnancy reactions, but caused thousands of cases of teratogenicity after taken by pregnant women, because the S-isomer not only has sedative effects but also has strong embryotoxicity and teratogenic effects (Blaschke G. et al., Arzneim-Forsch, 1979, 29(10), 1640-2). Therefore, it is necessary to resolve the pure enantiomers from the racemic mixture and study their properties, as the physiological differences between the enantiomers and the racemic mixture are unpredictable.

The inventors prepared the (-)-enantiomer (levoisomer or (R)-enantiomer) of 2-[(2-(4-fluorobenzoyl)phenyl) amino]-3-[(4-(2-carbazole-ethoxy)phenyl)]propionic acid and studied the pharmacological activity thereof.

## SUMMARY

The inventors of the present application attempted to prepare and obtained each of the enantiomers of 2-[(2-(4-fluorobenzoyl)phenyl)amino]-3-[(4-(2-carbazole-ethoxy)phenyl)]propionic acid and studied their pharmacological activities. It was unexpectedly found that the two isomers have significantly different pharmacological effects, wherein the levoisomer ((-)-enantiomer at a specific chiral position (see the position indicated by "*" in formula I) is significantly superior to (+)-enantiomer (ie. dextroisomer) in terms of activating the expression of RXR/PPAR-, RXR/PPAR- and RXR/PPAR-heterodimers and the hypoglycemic effect demonstrated in the db/db mouse model.

Based on the finding, in a first aspect, the present disclosure provides (-)-2-[(2-(4-fluorobenzoyl)phenyl) amino]-3-[(4-(2-carbazole-ethoxy)phenyl)]propionic acid or a pharmaceutically acceptable salt thereof that can selectively activate PPAR-α, PPAR-γ and PPAR-δ.

In a second aspect, the present disclosure provides a preparation method of (-)-2-[(2-(4-fluorobenzoyl)phenyl) amino]-3-[(4-(2-carbazole-ethoxy)phenyl)]propionic acid or a pharmaceutically acceptable salt thereof.

In a third aspect, the present disclosure provides (-)-2-[(2-(4-fluorobenzoyl)phenyl)amino]-3-[(4-(2-carbazole-ethoxy)phenyl)]propionic acid or a pharmaceutically acceptable salt thereof for use for treating a disease associated withthe regulation of RXR/PPAR, wherein the disease is selected from the group consisting of type 2 diabetes, lipid metabolism disorders, syndrome X, cardiovascular disease, coronary artery disease, hypercholesterolemia and obesity.

The chemical structure of the compound (-)-2-[(2-(4-fluorobenzoyl)phenyl)amino]-3-[(4-(2-carbazole-ethoxy) phenyl)]propionic acid according to the present disclosure is shown in formula (I):

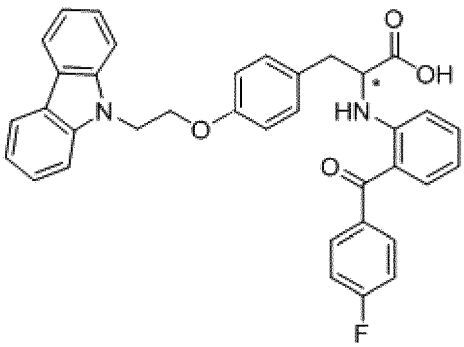

(I)

the position indicated by * a chiral position

Enantiomer 1 ((R)-Enantiomer):
(-)-2-[(2-(4-fluorobenzoyl)phenyl)amino]-3-[(4-(2-carbazole-ethoxy)phenyl)]propionic acid;

Enantiomer 2 ((S)-Enantiomer):
(+)-2-[(2-(4-fluorobenzoyl)phenyl)amino]-3-[(4-(2-carbazole-ethoxy)phenyl)]propionic acid

"Pharmaceutically acceptable salt" as used herein means a salt that is pharmaceutically acceptable. Bases used in the preparation of the salts include, but are not limited to, alkali metal (eg, sodium, potassium) hydroxides, alkaline earth metal (eg, calcium, magnesium) hydroxides, aqueous ammonia, and organic amines ($NR^1R^2R^3$, wherein $R^1$, $R^2$ and $R^3$ may be the same or different and preferably being hydrogen atom or a C1-C4 alkyl group).

"Pharmaceutically acceptable" as used herein is understood to be suitable for human and animal use within a reasonable medical range without excessive side effects including toxicity, allergic reactions, irritation and complications, and other deleterious effects.

The present invention also relates to a pharmaceutical composition comprising (-)-2-[(2-(4-fluorobenzoyl) phenyl)amino]-3-[(4-(2-carbazole-ethoxy)phenyl)]propionic acid or a pharmaceutically acceptable salt thereof, a pharmaceutical adjuvant including a pharmaceutically acceptable excipient and/or carrier. The (-)-2-[(2-(4-fluorobenzoyl) phenyl)amino]-3-[(4-(2-carbazole-ethoxy)phenyl)]propionic acid or a pharmaceutically acceptable salt thereof in the present disclosure may be used in combination with one or more other active pharmaceutical ingredients, which may be in any pharmaceutically acceptable combination.

The term "pharmaceutically acceptable excipient and/or carrier" as used herein means a non-toxic, inert, solid, semi-solid or liquid filler, diluent, encapsulating material or formulation aid of any type. Remington's Pharmaceutical Sciences Ed. by Gennaro, Mack Publishing, Easton, Pa., 1995, discloses various carriers for formulating pharmaceutical compositions and known techniques for preparing these pharmaceutical compositions. Some examples of materials that can be used as pharmaceutically acceptable carriers include, but are not limited to, sugars such as lactose, glucose, and sucrose; starches such as corn starch and potato starch; cellulose and its derivatives, such as sodium carboxymethylcellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; diols such as propylene glycol; esters such as ethyl oleate and ethyl laurate; agar; detergents such as TWEEN™ 80; buffers such as magnesium hydroxide and aluminum hydroxide; alginic acid; pyrogen-free water; isotonic saline; Ringer's solution; ethanol; and phosphate buffer solution and other non-toxic compatible lubricants, such as sodium lauryl sulfate and magnesium stearate; and colorants, coating agents, sweeteners, flavorings, and fragrances; preservatives and antioxidants may also be present in the compositions, as judged by the formulator. If filtration or other terminal sterilization processes are not feasible, the formulation can be produced under sterile conditions.

The (-)-2-[(2-(4-fluorobenzoyl)phenyl)amino]-3-[(4-(2-carbazole-ethoxy)phenyl)] propionic acid or a pharmaceutically acceptable salt thereof as described in the present invention can be prepared into various dosage forms with conventional pharmaceutical excipients, such as but not limited to oral preparations (tablets, capsules, powders, granules, syrups, pills, etc.), injection preparations, and topical preparations, etc.. The pharmaceutical composition of the present invention may usually contain 0.5 to 70% by weight of the active ingredient, preferably 1 to 20% by weight.

Advantageous effects of the present disclosure

In the present invention, (-)-2-[(2-(4-fluorobenzoyl)phenyl)amino]-3-[(4-(2-carbazole-ethoxy)phenyl)]propionic acid is separated from the 2-[(2-(4-fluorobenzoyl)phenyl) amine]-3-[(4-(2-carbazole-ethoxy)phenyl)]propionic acid enantiomers in the racemate, which activates the expression of RXR/PPAR-, RXR/PPAR-□and RXR/PPAR- heterodimers and exhibits a significantly better hypoglycemic effect in the db/db mouse model, further improving the therapeutic effect of [(2-(4-fluorobenzoyl)phenyl)amine]-3-[(4-(2-carbazole-ethoxy)phenyl)]propionic acid compounds on RXR or PPAR nuclear receptors associated diseases including type 2 diabetes, lipid metabolism disorders, syndrome X, cardiovascular disease, coronary artery disease, hypercholesterolemia and obesity.

## BRIEF DESCRIPTION OF DRAWINGS

Figure 1 shows the activation activity of enantiomers 1 and 2 of the present disclosure on PPARα in an *in vitro* reporter assay experimental model;

Figure 2 shows the activation activity of enantiomers 1 and 2 of the present disclosure on PPARγ in an *in vitro* reporter assay experimental model;

Figure 3 shows the activation activity of enantiomers 1 and 2 of the present disclosure on PPAR-δ in an *in vitro* reporter assay experimental model.

## DETAILED DESCRIPTION

The contents of the present disclosure are further described below with reference to examples. The percentages stated in the present disclosure are all percentages by weight unless otherwise specified. The range of values, such as units of measure or percentages, described in the specification are intended to provide an unambiguous written reference. The person skilled in the art will still be able to obtain the desired results based on the teachings and principles of the present disclosure, using temperatures, concentrations, amounts, etc. outside of this range or different from a single value.

Example 1: Preparation of enantiomer 1 and 2 of

[(2-(4-fluorobenzoyl)phenyl)amine]-3-[(4-(2-carbazole-ethoxy)phenyl)]propionic acid

20 g of mixture of enantiomers was dissolved in 2070 ml of methanol and applied to a supercritical chromatograph Chiralpak column (AY 20*250 mm). The column was first washed with a methanol/carbon dioxide mixture (volume ratio of 4:1) at a flow rate of 80 mL/min for 120 minutes, and then eluted with a methanol/carbon dioxide/aqueous ammonia mixture (volume ratio of 6:4:0.005) at a flow rate of 80 mL/min for 30 minutes. After about 6 minutes, an elution peak of the objective compound enantiomer 1 was obtained; after about 11 minutes, a peak of the enantiomer 2 was obtained. After evaporation of the solvent, enantiomer 1 (15 g) and enantiomer 2 (2 g) were obtained, respectively.

(-)-2-[(2-(4-fluorobenzoyl)phenyl)amino]-3-[(4-(2-carbazole-ethoxy)phenyl)]propionic acid (enantiomer 1):

$$\text{content}=100\%$$

$$\text{ee}\%=100\%$$

$$[\alpha]^{24}_{D} = -111.5\ (c=0.01,\ \text{DMSO})$$

Infrared spectrum (KBr, $cm^{-1}$): 3323, 3047, 2983, 2938, 2870, 2730, 2492, 1922, 1893, 1622, 1598, 1508, 1456, 1387, 1249, 927, 850, 751;
$^1$H-NMR (DMSO-$d_6$) $\delta$ 2.88 (dd, 1H, J=13.8, 6.1 Hz, one of $CH_2$), 3.06 (dd, 1H, J=13.8, 5 Hz, one of $CH_2$), 4.01-4.04 (m, H, CH), 4.25 (t, J=5.3 Hz, 2H, $CH_2$), 4.72 (t, J=5.3 Hz, 2H, $CH_2$), 6.44 (t, 1H, J=7.5 Hz, Ar-H), 6.60 (d, J=8.7 Hz, 2H, Ar-H), 6.70 (d, J=8.6 Hz, 1H, Ar-H), 7.00 (d, J=8.6 Hz, 2H, Ar-H), 7.19 (t, 2H, J=7.5 Hz, Ar-H), 7.24 (dd, 1H, J=8.1, 1.5 Hz, Ar-H), 7.26-7.32 (m, 3H, Ar-H), 7.43 (td, 2H, J=7.7, 1 Hz, Ar-H), 7.55-7.58 (m, 2H, Ar-H), 7.63 (d, J=8.3 Hz, 2H, Ar-H), 8.13 (d, J=7.8 Hz, 2H, Ar-H), 8.64 (d, J=7.0 Hz, 1H, NH);
HRMS ($C_{36}H_{29}FN_2O_4$) calcd. (%): 572.2111; found (%): 572.2108.

(+)-2-[(2-(4-fluorobenzoyl)phenyl)amino]-3-[(4-(2-carbazole-ethoxy)phenyl)]propionic acid (enantiomer 2):

$$\text{content}=100\%$$

$$\text{ee}\%=99.5\%$$

$$[\alpha]^{24}_{D} = 98.5\ (c=0.01,\ \text{DMSO})$$

Infrared spectrum (KBr, $cm^{-1}$): 3318, 3048, 2927, 2869, 2495, 1921, 1892, 1621, 1598, 1509, 1457, 1250, 1154, 927, 849, 751;

$^1$H-NMR (DMSO-$d_6$) $\delta$ 2.91 (dd, 1H, J=13.8, 6.2 Hz, one of $CH_2$), 3.06 (dd, 1H, J=13.8, 5 Hz, one of $CH_2$), 4.14-4.17 (m, 1H, CH), 4.24 (t, J=5.3 Hz, 2H, $CH_2$), 4.72 (t, J=5.3 Hz, 2H, $CH_2$), 6.47 (t, 1H, J=7.4 Hz, Ar-H), 6.61 (d, J=8.6 Hz, 2H, Ar-H), 6.73 (d, J=8.6 Hz, 1H, Ar-H), 7.01 (d, J=8.6 Hz, 2H, Ar-H), 7.18 (t, 2H, J=7.4 Hz, Ar-H), 7.26 (dd, 1H, J=8.0, 1.4 Hz, Ar-H), 7.29-7.32 (m, 3H, Ar-H), 7.42 (td, 2H, J=7.7, 0.9 Hz, Ar-H), 7.56-7.59 (m, 2H, Ar-H), 7.63 (d, J=8.3 Hz, 2H, Ar-H), 8.12 (d, J=7.7 Hz, 2H, Ar-H), 8.63 (d, J=7.1 Hz, 1H, NH);

HRMS ($C_{36}H_{29}FN_2O_4$) calcd. (%): 572.2111; found (%): 572.2109

Example 2: Preparation of sodium (-)-2-[(2-(4-fluorobenzoyl)phenyl)amino]-3-[(4-(2-carbazole-ethoxy)phenyl)]propionate (sodium salt of enantiomer 1)

30 ml of tetrahydrofuran was added into a 50 mL round bottom flask, and (-)-2-[(2-(4-fluorobenzoyl)phenyl) amino]-3-[(4-(2-carbazole-ethoxy)phenyl)]propionic acid (1000 mg, 1.75 mmol) was added with stirring, until dissolved. Sodium hydroxide (71 mg, 1.77 mmol) was dissolved in 2 mL of methanol. Then, the methanol solution of sodium hydroxide was added dropwise into (-)-2-[(2-(4-fluorobenzoyl)phenyl)amino]-3-[(4-(2-carbazole-ethoxy)phenyl)] propionic acid in tetrahydrofuran under stirring and the reaction was continued with stirring for 30 minutes. The mixture was distilled under reduced pressure and the distillation residue was dissolved in 2mL of dichloromethane. 30 ml of isopropyl ether was added into a 50 ml round bottom flask and the above dichloromethane solution was added dropwise with stirring. After the completion of the dropwise addition, the mixture was stirred for 2 minutes and filtered off with suction. The filter cake was rinsed with 5 ml of isopropyl ether, dried under vacuum at room temperature for 4 hours, and dried under vacuum at 60 °C to a loss on drying less than 0.5% by weight to give a yellow solid sodium (-)-2-[(2-(4-fluorobenzoyl)phenyl) amino]-3-[(4-(2-carbazole-ethoxy)phenyl)]propionate (1g, 1.68 mmol, 96.3% yield).

$$content=98.7\%$$

$$ee\%=96.1\%$$

$$[\alpha]^{24}{}_{D} = = -119.5\ (c=0.01,\ DMSO)$$

Infrared spectrum (KBr, $cm^{-1}$): 3352, 3051, 2969, 2930, 2871, 1921, 1891, 1617, 1598, 1509, 1457, 1400, 1242, 928, 850, 750;
X-ray powder diffraction: amorphous

The measured data and analytical results of NMR (Bruker AVANCE III HD 500, DMSO-$d_6$), including 1-H NMR, 13-C NMR, COSY, HMQC and HMBC are shown in Table 1:

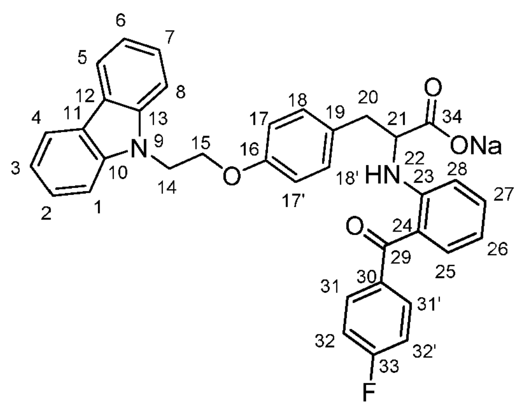

Table 1 the data and analysis of NMR spectra of sodium (-)-2-[(2-(4-fluorobenzoyl)phenyl)amino]-3-[(4-(2-carbazole-ethoxy)phenyl)]propionate

| Atomic number | Chemical shift δH (ppm) | Chemical shift δC (ppm) | COSY 1H-1H | HSQC Homo 13C-1H | HMBC Hetero 13C-1H |
|---|---|---|---|---|---|
| 1(8) | 7.63 (d, 2H, J=8.3 Hz) | 109.56 | 7.63 (7.41-7.45)<br>7.63 (7.17-7.19) | 109.56 (7.63) | 109.56 (7.41-7.45, 7.17-7.19, 8.13) |
| | | | 7.41-7.45 (7.17-7.19) | | |

(continued)

| Atomic number | Chemical shift δH (ppm) | Chemical shift δC (ppm) | COSY 1H-1H | HSQC Homo 13C-1H | HMBC Hetero 13C-1H |
|---|---|---|---|---|---|
| 2(7) | 7.41-7.45 (m, 2H) | 125.6 | 7.41-7.45 (7.63)<br>7.41-7.45 (8.13) | 125.60 (7.41-7.45) | 125.60 (8.13, 7.17-7.19) |
| 3(6) | 7.17-7.19 (m, 2H) | 118.83 | 7.17-7.19 (7.41-7.45)<br>7.17-7.19 (8.13)<br>7.17-7.19 (7.63) | 118.83 (7.17-7.19) | 118.83 (7.63) |
| 4(5) | 8.13 (d, 2H, J=7.7 Hz) | 120.09 | 8.13 (7.17-7.19)<br>8.13 (7.41-7.45) | 120.09 (8.13) | 120.09 (7.41-7.45) |
| 10(13) | | 140.21 | | | 140.21 (4.73, 7.41-7.45, 8.13) |
| 11(12) | | 122.1 | | | 122.10 (7.17-7.19, 7.63, 8.13) |
| 14 | 4.73 (t, 2H, J=5.3 Hz) | 42.08 | 4.73 (4.24) | 42.08 (4.73) | 42.08 (4.24) |
| 15 | 4.24 (t, 2H, J=5.4 Hz) | 66.17 | 4.24 (4.73) | 66.17 (4.24) | 66.17 (4.73) |
| 16 | | 156.23 | | | 156.23 (7.01, 6.59, 4.23) |
| 17(17') | 6.59 (d, 2H, J=8.7 Hz) | 113.64 | 6.59 (7.01) | 113.64 (6.59) | 113.64 (7.01) |
| 18(18') | 7.01 (d, 2H, J=8.6 Hz) | 130.35 | 7.01 (6.59) | 130.35 (7.01) | 130.35 (2.88, 3.06, 6.59) |
| 19 | | 131.51 | | | 131.51 (2.88,3.06, 3.90-3.93, 6.59) |
| 20 | 2.88 (dd, 1H, J=13.7, 5.9 Hz)<br>3.06 (dd, 1H, J=13.7, 5.1 Hz) | 37.13 | 2.88 (3.90-3.93)<br>3.06 (3.90-3.93) | 37.13 (2.88, 3.06) | 37.13 (3.90-3.93, 7.01) |
| 21 | 3.90-3.93 (m, 1H) | 59 | 3.90-3.93 (2.88)<br>3.90-3.93 (3.06)<br>3.90-3.93 (8.74) | 59 (3.90-3.93) | 59.00 (2.88, 3.06, 8.74) |
| 22 | 8.74 (d, 1H, J=6.7 Hz) | | 8.74 (3.90-3.93) | | |
| 23 | | 150.31 | | | 150.31 (7.20-7.26, 3.90-3.93) |
| 24 | | 116.5 | | | 116.50 (6.39, 6.66, 8.74) |
| 25 | 7.20-7.26 (m, 1H) | 134.22 | 7.20-7.26 (6.39) | 134.22 (7.20-2.26) | 134.22 (7.20-7.26) |
| 26 | 6.39 (t, 1H, J=7.3 Hz) | 112.48 | 6.39 (7.20-7.26)<br>6.39 (7.20-7.26) | 112.48 (6.39) | 112.48 (6.66) |
| 27 | 7.20-7.26 (m, 1H) | 134.48 | 7.20-7.26 (6.39)<br>7.20-7.26 (6.66) | 134.48 (7.20-7.26) | 134.48 (7.20-7.26, 6.39) |

(continued)

| Atomic number | Chemical shift δH (ppm) | Chemical shift δC (ppm) | COSY 1H-1H | HSQC Homo 13C-1H | HMBC Hetero 13C-1H |
|---|---|---|---|---|---|
| 28 | 6.66 (d, 1H, J=8.6 Hz) | 112.63 | 6.66 (7.20-7.26) | 112.63 (6.66) | 112.63 (6.39, 8.74) |
| 29 | | 195.72 | | | 195.72 (7.55-7.58, 7.20-7.26) |
| 30 | | 136.8 | | | 136.80 (7.28-7.32) |
| 31(31') | 7.55-7.58 (m, 2H) | 131.25 3JF-C=8.8 | 7.55-7.58 (7.28-7.32) | 131.25 (7.55-7.58) | |
| 32(32') | 7.28-7.32 (m, 2H) | 115.1 2JF-C=22.5 | 7.28-7.32 (7.55-7.58) | 115.10 (7.28-7.32) | 115.10 (7.55-7.58) |
| 33 | | 163.4 1JF-C=248 | | | 163.40 (7.55-7.58, 7.28-7.32) |
| 34 | | 173.67 | | | 173.67 (2.88, 3.06, 3.90-3.93, 8.74) |
| FAB-MS (m/z): 595(M+1) | | | | | |

Example 3: Preparation of sodium (+)-2-[(2-(4-fluorobenzoyl)phenyl)amine]-3-[(4-(2-carbazole-ethoxy)phenyl)]propionate (sodium salt of enantiomer 2)

30 ml of tetrahydrofuran was added into a 50 mL round bottom flask, and (+)-2-[(2-(4-fluorobenzoyl)phenyl) amino]-3-[(4-(2-carbazole-ethoxy)phenyl)]propionic acid (1000 mg, 1.75 mmol) was added with stirring, until dissolved. Sodium hydroxide (71 mg, 1.77 mmol) was dissolved in 2 mL of methanol. Then the methanol solution of sodium hydroxide was added dropwise into (+)-2-[(2-(4-fluorobenzoyl)phenyl)amino]-3-[(4-(2-carbazole-ethoxy) phenyl)]propionic acid in tetrahydrofuran under stirring and the reaction was continued with stirring for 30 minutes. The mixture was distilled under reduced pressure and the distillation residue was dissolved in 2mL of dichloromethane. 30 ml of isopropyl ether was added into a 50 ml round bottom flask and the above dichloromethane solution was added dropwise with stirring. After the completion of the dropwise addition, the mixture was stirred for 2 minutes and filtered off with suction. The filter cake was rinsed with 5 ml of isopropyl ether, dried under vacuum at room temperature for 4 hours, and dried under vacuum at 60 °C to a loss on drying less than 0.5% by weight to give a yellow solid sodium (+)-2-[(2-(4-fluorobenzoyl)phenyl)amino]-3-[(4-(2-carbazole-ethoxy)phenyl)]propionate (980mg, 1.65 mmol, 94.1% yield).

$$\text{content}=97.3\%$$

$$\text{ee}\%=98.4\%$$

$$[\alpha]^{24}_{D} = = 128.8\ (c=0.01,\ DMSO)$$

Infrared spectrum (KBr, $cm^{-1}$): 3354, 3051, 2969, 2929, 2871, 1922, 1891, 1617, 1598, 1509, 1401, 1243, 928, 850, 750;

X-ray powder diffraction: amorphous

The measured data and analytical results of NMR (Bruker AVANCE III HD 500, DMSO-$d_6$), including 1-H NMR, 13-C NMR, COSY, HMQC and HMBC are shown in Table 2:

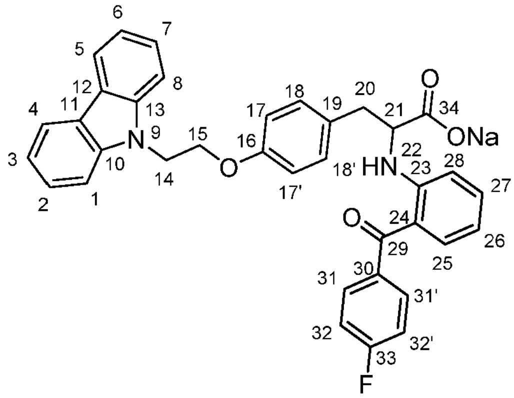

Table 2 the data and analysis of NMR spectra of sodium (+)-2-[(2-(4-fluorobenzoyl)phenyl)amine]-3-[(4-(2-carbazole-ethoxy)phenyl)]propionate

| Atomic number | Chemical shift δH (ppm) | Chemical shift δC (ppm) | COSY 1H-1H | HSQC Homo13C-1 H | HMBC Hetero 13C-1H |
|---|---|---|---|---|---|
| 1(8) | 7.63 (d, 2H, J=8.3 Hz) | 109.55 | 7.63 (7.41-7.45)<br>7.63 (7.17-7.19) | 109.55 (7.63) | 109.55 (7.41-7.45, 7.17-7.19, 8.13) |
| 2(7) | 7.41-7.45 (m, 2H) | 125.59 | 7.41-7.45 (7.17-7.19)<br>7.41-7.45 (7.63)<br>7.41-7.45 (8.13) | 125.59 (7.41-7.45) | 125.59 (8.13, 7.17-7.19) |
| 3(6) | 7.17-7.19 (m, 2H) | 118.83 | 7.17-7.19 (7.41-7.45)<br>7.17-7.19 (8.13)<br>7.17-7.19 (7.63) | 118.83 (7.17-7.19) | 118.83 (7.63) |
| 4(5) | 8.13 (d, 2H, J=7.7 Hz) | 120.09 | 8.13 (7.17-7.19)<br>8.13 (7.41-7.45) | 120.09 (8.13) | 120.09 (7.41-7.45) |
| 10(13) | | 140.21 | | | 140.21 (4.73, 7.41-7.45, 8.13) |
| 11(12) | | 122.1 | | | 122.10 (7.17-7.19, 7.63, 8.13) |
| 14 | 4.73 (t, 2H, J=5.3 Hz) | 42.07 | 4.73 (4.24) | 42.07 (4.73) | 42.07 (4.24) |
| 15 | 4.24 (t, 2H, J=5.4 Hz) | 66.17 | 4.24 (4.73) | 66.17 (4.24) | 66.17 (4.73) |
| 16 | | 156.23 | | | 156.23 (7.01, 6.59, 4.24) |
| 17(17’) | 6.59 (d, 2H, J=8.7 Hz) | 113.64 | 6.59 (7.01) | 113.64 (6.59) | 113.64 (7.01) |
| 18(18’) | 7.01 (d, 2H, J=8.7 Hz) | 130.35 | 7.01 (6.59) | 130.35 (7.01) | 130.35 (2.88, 3.06, 6.59) |
| 19 | | 131.5 | | | 131.50 (2.88,3.06, 3.90-3.93, 6.59) |

(continued)

| Atomic number | Chemical shift δH (ppm) | Chemical shift δC (ppm) | COSY 1H-1H | HSQC Homo13C-1 H | HMBC Hetero 13C-1H |
|---|---|---|---|---|---|
| 20 | 2.88 (dd, 1H, J=13.7, 5.9 Hz) | 37.13 | 2.88 (3.90-3.93) | 37.13 (2.88, 3.06) | 37.13 (3.90-3.93, 7.01) |
| | 3.07 (dd, 1H, J=13.7, 5.1 Hz) | | 3.06 (3.90-3.93) | | |
| 21 | 3.90-3.93 (m, 1H) | 58.99 | 3.90-3.93 (2.88)<br>3.90-3.93 (3.06) 3.90-3.93 (8.74) | 58.99 (3.90-3.93) | 58.99 (2.88, 3.06, 8.74) |
| 22 | 8.74 (d, 1H, J=6.8 Hz) | | 8.74 (3.90-3.93) | | |
| 23 | | 150.3 | | | 150.30 (7.20-7.26, 3.90-3.93) |
| 24 | | 116.5 | | | 116.50 (6.39, 6.66, 8.74) |
| 25 | 7.20-7.26 (m, 1H) | 134.22 | 7.20-7.26 (6.39) | 134.22 (7.20-2.26) | 134.22 (7.20-7.26) |
| 26 | 6.39 (t, 1H, J=7.5 Hz) | 112.48 | 6.39 (7.20-7.26)<br>6.39 (7.20-7.26) | 112.48 (6.39) | 112.48 (6.66) |
| 27 | 7.20-7.26 (m, 1H) | 134.48 | 7.20-7.26 (6.39)<br>7.20-7.26 (6.66) | 134.48 (7.20-7.26) | 134.48 (7.20-7.26, 6.39) |
| 28 | 6.66 (d, 1H, J=8.6 Hz) | 112.63 | 6.66 (7.20-7.26) | 112.63 (6.66) | 112.63 (6.39, 8.74) |
| 29 | | 195.73 | | | 195.73 (7.55-7.58, 7.20-7.26) |
| 30 | | 136.8 | | | 136.80 (7.28-7.32) |
| 31(31') | 7.55-7.58 (m, 2H) | 131.25<br>3JF-C=8.8 | 7.55-7.58 (7.28-7.32) | 131.25 (7.55-7.58) | |
| 32(32') | 7.28-7.32 (m, 2H) | 115.1<br>2JF-C=21.3 | 7.28-7.32 (7.55-7.58) | 115.10 (7.28-7.32) | 115.10 (7.55-7.58) |
| 33 | | 163.4<br>1JF-C=248 | | | 163.40 (7.55-7.58, 7.28-7.32) |
| 34 | | 173.71 | | | 173.71 (2.88, 3.06, 3.90-3.93, 8.74) |
| **[0057]** FAB-MS (m/z): 595(M+1) | | | | | |

Example 4 (-)-2-[(2-(4-fluorobenzoyl)phenyl)amino]-3-[(4-(2-carbazole-ethoxy)phenyl)]propionic acid ( Enantiomer 1) exhibited activity of PPARα activator in an *in vitro* reporter assay model experiment

The PPARα reporter gene detection system comprises a pCDNA3.1 luciferase expression plasmid (PPRE enhancer element was inserted into the upstream of the promoter), a green fluorescent protein GFP expression plasmid, and a human PPARα expression plasmid.

The above plasmids were co-transfected (Fugene 6.0 transfection reagent) into human hepatoma cell line L-02. 48 hours after transfection, different concentrations of test compounds were added. 24 hours after administration, the cells were treated with a luciferase assay kit (Promega E1910) and the fluorescence intensity of GFP (wavelength 485-527nm) and luciferase substrate (wavelength 562nm) were detected using a fluorescence detector (Fluoroskan Ascent FL)

respectively. The GFP signal was used as an internal reference to correct the detected luciferase substrate signal, and a relative reporter activation intensity was obtained by comparing the above corrected signal to the blank control (without treatment of the compounds). The semi-activating activity (EC50) of the test compound was calculated from concentration gradient data.

Compared to enantiomer 2, enantiomer 1 has a more significant effect on activating PPARα, with $EC_{50}$ of 1.135 μM and 5.385 μM, respectively (see Figure 1).

Example 5 (-)-2-[(2-(4-fluorobenzoyl)phenyl)amino]-3-[(4-(2-carbazole-ethoxy)phenyl)]propionic acid ( Enantiomer 1) exhibited activity of RXR/PPARγ heterodimer activator in an *in vitro* reporter assay model experiment

The PPARγ reporter gene detection system comprises a pACOX fluorescent reporter gene plasmid (the promoter comprises PPRE sequence), PPARγ expression plasmid, RXR expression plasmid, and a green fluorescent protein GFP expression plasmid.

The above plasmids were co-transfected (Fugene 6.0 transfection reagent) into human hepatoma cell line L-02. 48 hours after transfection, different concentrations of test compounds were added. 24 hours after administration, the cells were treated with a luciferase assay kit (Promega E1910) and the fluorescence intensity of GFP (wavelength 485-527nm) and luciferase substrate (wavelength 562nm) were detected using a fluorescence detector (Fluoroskan Ascent FL), respectively. The GFP signal was used as an internal reference to correct the detected luciferase substrate signal, and a relative reporter activation intensity was obtained by comparing the above corrected signal to the blank control (without treatment of the compounds). The semi-activating activity (EC50) of the test compound was calculated from concentration gradient data.

Compared to enantiomer 2, enantiomer 1 has a more significant effect on activating PPARγ, with $EC_{50}$ of 0.076 μM and 2.709 μM, respectively (see Figure 2).

Example 6 (-)-2-[(2-(4-fluorobenzoyl)phenyl)amino]-3-[(4-(2-carbazole-ethoxy)phenyl)]propionic acid ( Enantiomer 1) exhibited activity of RXR/PPAR-δ heterodimer activator in an *in vitro* reporter assay model experiment

The PPARδ reporter gene detection system comprises a pGL3-PPRE fluorescent reporter gene plasmid (PPRE enhancer element was inserted into the upstream of the promoter), PPARδ expression plasmid, RXR expression plasmid, and a green fluorescent protein GFP expression plasmid.

The above plasmids were co-transfected (Fugene 6.0 transfection reagent) into human hepatoma cell line L-02. 48 hours after transfection, different concentrations of test compounds were added. 24 hours after administration, the cells were treated with a luciferase assay kit (Promega E1910) and the fluorescence intensity of GFP (wavelength 485-527nm) and luciferase substrate (wavelength 562nm) were detected using a fluorescence detector (Fluoroskan Ascent FL), respectively. The GFP signal is used as an internal reference to correct the detected luciferase substrate signal, and a relative reporter activation intensity was obtained by comparing the above corrected signal to the blank control (without treatment of the compounds). The semi-activating activity ($EC_{50}$) of the test compound was calculated from concentration gradient data.

Compared to enantiomer 2, enantiomer 1 has a more significant effect on activating PPAR-δ, with $EC_{50}$ of 1.93 μM and >10 μM (no remarkable activity determined under the highest concentration), respectively (see Figure 3).

Example 7 (-)-2-[(2-(4-fluorobenzoyl)phenyl)amino]-3-[(4-(2-carbazole-ethoxy)phenyl)]propionic acid ( Enantiomer 1) was effective in reducing blood glucose concentrations in db/db mice.

The experiment consisted of 4 groups: a model group, two administration groups (administered with enantiomers 1 and 2, respectively) and a positive control group (rosiglitazone), with 10 mice per group. The route of administration was oral, the dose was 20 mg/kg in the administration group and 5 mg/kg in the control group, the frequency of administration was once a day, and the administration period was 14 days. The animals of groups 1 to 4 were orally administered with vehicle, enantiomer 1, enantiomer 2 and the positive drug rosiglitazone, respectively. On days 0, 3, 6, 9, 12, and 14, after fasting for 6 hours after administration, blood was collected from the tail vein and fasting blood glucose levels was measured by a blood glucose meter and recorded. The results are shown in Table 3.

Table 3 the effect of (-)-2-[(2-(4-fluorobenzoyl)phenyl)amino]-3-[(4-(2-carbazole-ethoxy)phenyl)]propionic acid on blood glucose concentrations in mice

| Group | | Day 0 | Day 3 | Day 6 | Day 9 | Day 12 | Day 14 |
|---|---|---|---|---|---|---|---|
| GI:model group (n=10) | Average value | 18.98 | 25.5 | 23.69 | 26.49 | 29.23 | 18.52 |
| | SEM | 1.47 | 1.12 | 1.17 | 0.97 | 0.99 | 1.28 |

(continued)

| Group | | Day 0 | Day 3 | Day 6 | Day 9 | Day 12 | Day 14 |
|---|---|---|---|---|---|---|---|
| G2: enantiomer 1(n=10) | Average value | 18.43 | 18.2 | 14.7 | 18.14 | 16.18 | 10.98 |
| | SEM | 1.35 | 1.87 | 1.33 | 1.42 | 1.48 | 0.83 |
| G3: Enantiomer 2 (n=10) | Average value | 18.86 | 23.99 | 20.03 | 26.01 | 26.44 | 17.18 |
| | SEM | 1.28 | 1.38 | 1.5 | 1.45 | 1.17 | 1.09 |
| G4: rosiglitazone (n=10) | Average value | 19.09 | 13.58 | 13.8 | 15.05 | 14.44 | 11.29 |
| | SEM | 1.36 | 1 | 0.82 | 0.86 | 0.46 | 1.09 |

Example 8 (-)-2-[(2-(4-fluorobenzoyl)phenyl)amino]-3-[(4-(2-carbazole-ethoxy)phenyl)]propionic acid ( Enantiomer 1) increased glucose tolerance in the glucose tolerance test (OTGG)

After 14 days of administration, the animals were fasted overnight (<10 hours), and orally given 2 g/kg of glucose on the next day. Blood was collected from the tail vein at the 0 (pre-administration or fasting blood glucose level), 15, 30, 60 and 120 minutes after administration, respectively, and blood glucose levels were then measured by blood glucose meter and recorded. The results are shown in Table 4

Table 4 the effect of (-)-2-[(2-(4-fluorobenzoyl)phenyl)amino]-3-[(4-(2-carbazole-ethoxy)phenyl)] propionic acid on glucose tolerance

| Group | | 0 min | 15min | 30min | 60min | 120min | AUC |
|---|---|---|---|---|---|---|---|
| Gl:model group (n=10) | Average value | 18.52 | 32.3 | 33.3 | 32.28 | 27.77 | 60.97 |
| | SEM | 1.28 | 0.72 | 0 | 0.58 | 1.96 | 1.54 |
| G2: enantiomer 1(n=10) | Average value | 10.98 | 28.02 | 32.26 | 26.6 | 14.69 | 47.77 |
| | SEM | 0.83 | 1.34 | 0.78 | 2.15 | 1.13 | 2.33 |
| G3: Enantiomer 2 (n=10) | Average value | 17.18 | 31.87 | 33.3 | 30.94 | 25.75 | 59.16 |
| | SEM | 1.09 | 1.43 | 0 | 1.3 | 1.8 | 1.59 |
| G4: rosiglitazone (n=10) | Average value | 11.29 | 28.11 | 31.22 | 25.08 | 13.61 | 45.76 |
| | SEM | 1.09 | 2.03 | 1.11 | 1.33 | 1.48 | 2.01 |

## Claims

1. (-)-2-[(2-(4-fluorobenzoyl)phenyl)amino]-3-[(4-(2-carbazole-ethoxy)phenyl)]propionic acid or a pharmaceutically acceptable salt thereof.

2. The (-)-2-[(2-(4-fluorobenzoyl)phenyl)amino]-3-[(4-(2-carbazole-ethoxy)phenyl)] propionic acid or a pharmaceutically acceptable salt thereof according to claim 1, wherein the salt is an alkali metal salt, an alkaline earth metal salt, an ammonium salt or a quaternary ammonium salt.

3. The (-)-2-[(2-(4-fluorobenzoyl)phenyl)amino]-3-[(4-(2-carbazole-ethoxy)phenyl)] propionic acid or a pharmaceutically acceptable salt thereof according to claim 2, wherein the alkali metal salt is a sodium or potassium salt, and/or the alkaline earth metal salt is a calcium or magnesium salt.

4. A supercritical chromatography method for preparing the (-)-2-[(2-(4-fluorobenzoyl)phenyl)amino]-3-[(4-(2-carbazole-ethoxy)phenyl)]propionic acid according to claim 1, comprising the steps of :

   dissolving 2-[(2-(4-fluorobenzoyl)phenyl)amino]-3-[(4-(2-carbazole-ethoxy)phenyl)] propionic acid in a solvent, loading the solution to a chiral chromatography column, eluting sequentially with a solvent mixture of organic solvent/carbon dioxide and a solvent mixture of organic solvent/carbon dioxide/aqueous ammonia and separating, to obtain

(-)-2-[(2-(4-fluorobenzoyl)phenyl)amino]-3-[(4-(2-carbazole-ethoxy)phenyl)]propionic acid.

5. (-)-2-[(2-(4-fluorobenzoyl)phenyl)amino]-3-[(4-(2-carbazole-ethoxy)phenyl)] propionic acid or a pharmaceutically acceptable salt for use in treating and preventing a disease associated with the regulation of RXR/PPAR, wherein the disease is selected from the group consisting of type 2 diabetes, lipid metabolism disorders, syndrome X, cardiovascular disease, coronary artery disease, hypercholesterolemia and obesity.

6. A pharmaceutical composition, comprising the (-)-2-[(2-(4-fluorobenzoyl)phenyl)amino]-3-[(4-(2-carbazole-ethoxy) phenyl)]propionic acid or a pharmaceutically acceptable salt thereof according to any one of claims 1 to 3, and a pharmaceutically acceptable excipient and/or carrier.

7. The pharmaceutical composition according to claim 6, wherein the content of (-)-2-[(2-(4-fluorobenzoyl)phenyl) amino]-3-[(4-(2-carbazole-ethoxy)phenyl)]propionic acid or a pharmaceutically acceptable salt thereof is 0.5-70% by weight.

8. The pharmaceutical composition according to claim 6 or 7, wherein said pharmaceutical composition is in the form of an oral formulation, injection preparation or topical preparation.

9. The pharmaceutical composition according to claim 8, wherein the composition is in the form of a tablet, capsule, powder, granule, syrup or pill.

**Patentansprüche**

1. (-)-2-[(2-(4-Fluorbenzoyl)phenyl)amino]-3-[(4-(2-carbazol-ethoxy)phenyl)]propionsäure oder pharmazeutisch annehmbares Salz davon.

2. (-)-2-[(2-(4-Fluorbenzoyl)phenyl)amino]-3-[(4-(2-carbazol-ethoxy)phenyl)]propionsäure oder pharmazeutisch annehmbares Salz davon nach Anspruch 1, wobei das Salz ein Alkalimetallsalz, ein Erdalkalimetallsalz, ein Ammoniumsalz oder ein quaternäres Ammoniumsalz ist.

3. (-)-2-[(2-(4-Fluorbenzoyl)phenyl)amino]-3-[(4-(2-carbazol-ethoxy)phenyl)]propionsäure oder pharmazeutisch annehmbares Salz davon nach Anspruch 2, wobei das Alkalimetallsalz ein Natrium- oder Kaliumsalz ist und/oder das Erdalkalimetallsalz ein Calcium- oder Magnesiumsalz ist.

4. Überkritisches Chromatographieverfahren zum Herstellen der (-)-2-[(2-(4-Fluorbenzoyl)phenyl)amino]-3-[(4-(2-carbazol-ethoxy)phenyl)]propionsäure nach Anspruch 1, umfassend die Folgenden Schritte:

Lösen von 2-[(2-(4-Fluorbenzoyl)phenyl)amino]-3-[(4-(2-Carbazol-ethoxy)phenyl)]propionsäure in einem Lösungsmittel, Laden der Lösung auf eine chirale Chromatographiesäule, sequentielles Eluieren mit einem Lösungsmittelgemisch aus organischem Lösungsmittel/Kohlendioxid und einem Lösungsmittelgemisch aus organischem Lösungsmittel/Kohlendioxid/wässrigem Ammoniak und Trennen, um
(-)-2-[(2-(4-Fluorbenzoyl)phenyl)amino]-3-[(4-(2-carbazol-ethoxy)phenyl)]propionsäure zu erlangen.

5. (-)-2-[(2-(4-Fluorbenzoyl)phenyl)amino]-3-[(4-(2-carbazol-ethoxy)phenyl)]propionsäure oder pharmazeutisch annehmbares Salz zur Verwendung bei der Behandlung und Prävention einer Krankheit, die mit der Regulierung von RXR/PPAR assoziiert ist, wobei die Krankheit ausgewählt ist aus der Gruppe, bestehend aus Typ-2-Diabetes, Fettstoffwechselstörungen, Syndrom X, kardiovaskulären Erkrankungen, Koronararterienerkrankungen, Hypercholesterinämie und Fettleibigkeit.

6. Pharmazeutische Zusammensetzung, umfassend die (-)-2-[(2-(4-Fluorbenzoyl)phenyl)amino]-3-[(4-(2-carbazolethoxy)phenyl)]propionsäure oder pharmazeutisch annehmbares Salz davon nach einem der Ansprüche 1 bis 3 und einen pharmazeutisch annehmbaren Hilfsstoff und/oder Träger.

7. Pharmazeutische Zusammensetzung nach Anspruch 6, wobei der Gehalt an (-)-2-[(2-(4-Fluorbenzoyl)phenyl)amino]-3-[(4-(2-carbazol-ethoxy)phenyl)]propionsäure oder einem pharmazeutisch annehmbaren Salz davon 0,5-70 Gewichts-% ist.

8. Pharmazeutische Zusammensetzung nach Anspruch 6 oder 7, wobei die pharmazeutische Zusammensetzung in Form einer oralen Formulierung, eines Injektionspräparats oder eines topischen Präparats ist.

9. Pharmazeutische Zusammensetzung nach Anspruch 8, wobei die Zusammensetzung in Form einer Tablette, Kapsel, eines Pulvers, Granulats, Sirups oder einer Pille ist.

## Revendications

1. L'acide (-)-2-[(2-(4-fluorobenzoyl)phényl)amino]-3-[(4-(2-carbazole-éthoxy)phényl)]acide propionique ou un de ses sels pharmaceutiquement acceptables.

2. L'acide (-)-2-[(2-(4-fluorobenzoyl)phényl)amino]-3-[(4-(2-carbazole-éthoxy)phényl)]propionique ou un de ses sels pharmaceutiquement acceptables selon la revendication 1, dans lequel le sel est un sel de métal alcalin, un sel de métal alcalino-terreux, un sel d'ammonium ou un sel d'ammonium quaternaire.

3. L'acide (-)-2-[(2-(4-fluorobenzoyl)phényl)amino]-3-[(4-(2-carbazole-éthoxy)phényl)]propionique ou un de ses sels pharmaceutiquement acceptables selon la revendication 2, dans lequel le sel de métal alcalin est un sel de sodium ou de potassium, et/ou le sel de métal alcalino-terreux est un sel de calcium ou de magnésium.

4. Procédé de chromatographie supercritique pour la préparation de l'acide (-)-2-[(2-(4-fluorobenzoyl)phényl)amino]-3-[(4-(2-carbazole-éthoxy)phényl)]propionique selon la revendication 1, comprenant les étapes suivantes :

   dissolution de l'acide 2-[(2-(4-fluorobenzoyl)phényl)amino]-3-[(4-(2-carbazole-éthoxy)phényl)] propionique dans un solvant, chargement de la solution dans une colonne de chromatographie chirale, élution séquentielle avec un mélange de solvant organique/dioxyde de carbone et un mélange de solvant organique/dioxyde de carbone/ammoniaque aqueuse et séparation, pour obtenir
   un acide (-)-2-[(2-(4-fluorobenzoyl)phényl)amino]-3-[(4-(2-carbazole-éthoxy)phényl)]propionique.

5. Un acide (-)-2-[(2-(4-fluorobenzoyl)phényl)amino]-3-[(4-(2-carbazole-éthoxy)phényl)]propionique ou un sel pharmaceutiquement acceptable pour le traitement et la prévention d'une maladie associée à la régulation de RXR/PPAR, la maladie étant sélectionnée dans le groupe constitué par le diabète de type 2, les troubles du métabolisme lipidique, le syndrome X, les maladies cardiovasculaires, les maladies coronariennes, l'hypercholestérolémie et l'obésité.

6. Composition pharmaceutique comprenant l'acide (-)-2-[(2-(4-fluorobenzoyl)phényl)amino]-3-[(4-(2-carbazole-éthoxy)phényl)]propionique ou un de ses sels pharmaceutiquement acceptables selon l'une quelconque des revendications 1 à 3, et un excipient et/ou un support pharmaceutiquement acceptable.

7. Composition pharmaceutique selon la revendication 6, dans laquelle la teneur en acide (-)-2-[(2-(4-fluorobenzoyl) phényl)amino]-3-[(4-(2-carbazole-ethoxy)phényl)]propionique ou en un sel pharmaceutiquement acceptable de celui-ci est de 0,5 à 70 % en poids.

8. Composition pharmaceutique selon la revendication 6 ou 7, dans laquelle ladite composition pharmaceutique se présente sous la forme d'une formulation orale, d'une préparation injectable ou d'une préparation topique.

9. Composition pharmaceutique selon la revendication 8, dans laquelle la composition se présente sous la forme d'un comprimé, d'une gélule, d'une poudre, d'un granulé, d'un sirop ou d'une pilule.

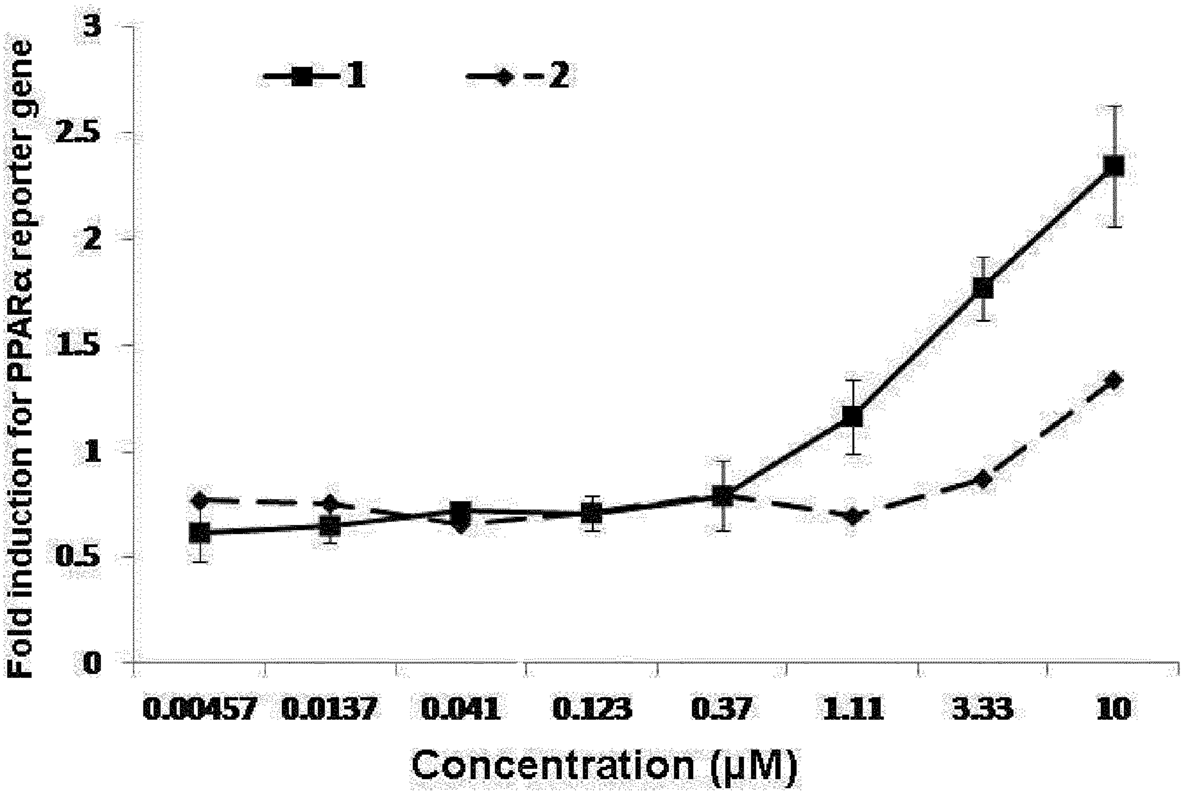
Fold induction for PPARα reporter gene
3
2.5
2
1.5
1
0.5
0
1
2
0.00457
0.0137
0.041
0.123
0.37
1.11
3.33
10
Concentration (μM)

Figure 1

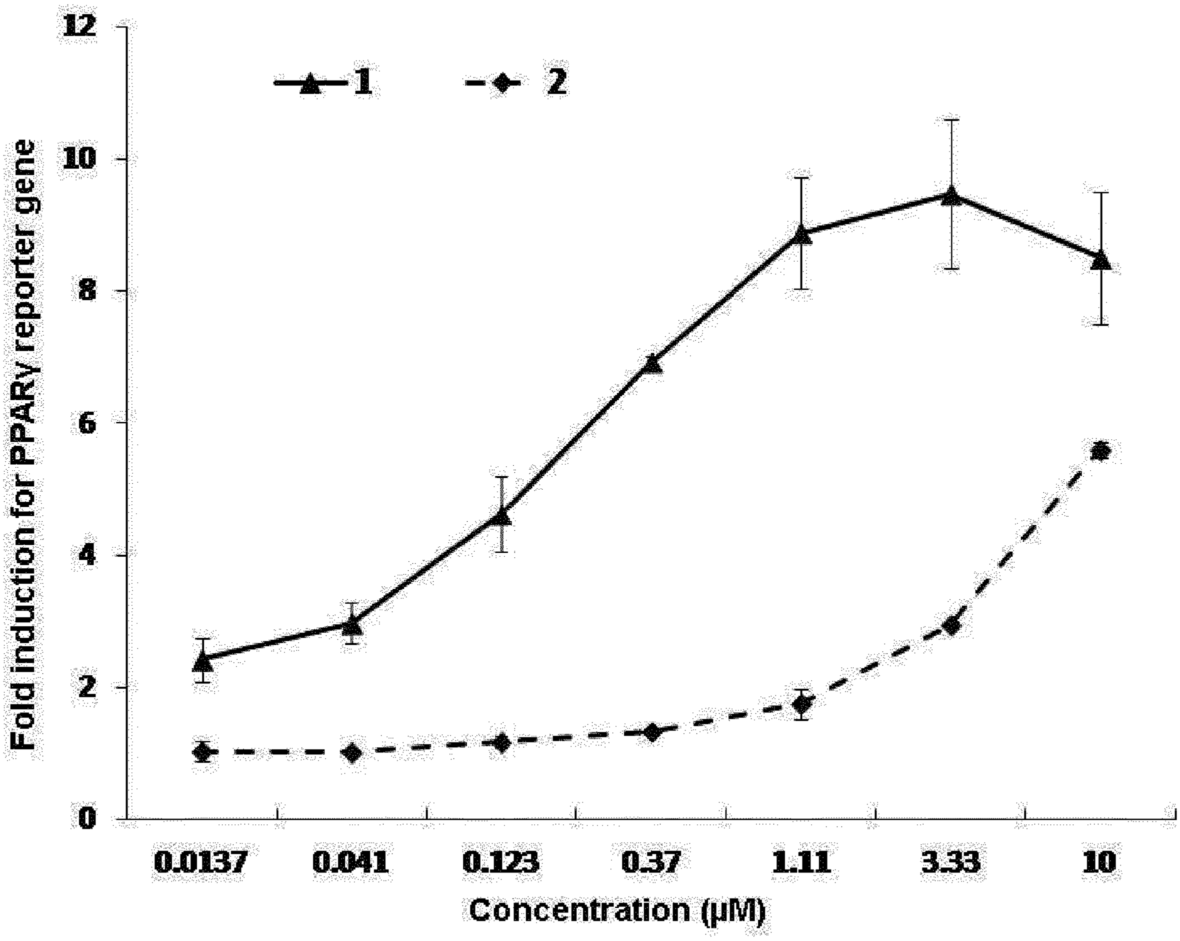
12
10
8
6
4
2
0
1
2
Fold induction for PPARγ reporter gene
0.0137
0.041
0.123
0.37
1.11
3.33
10
Concentration (μM)

**Figure 2**

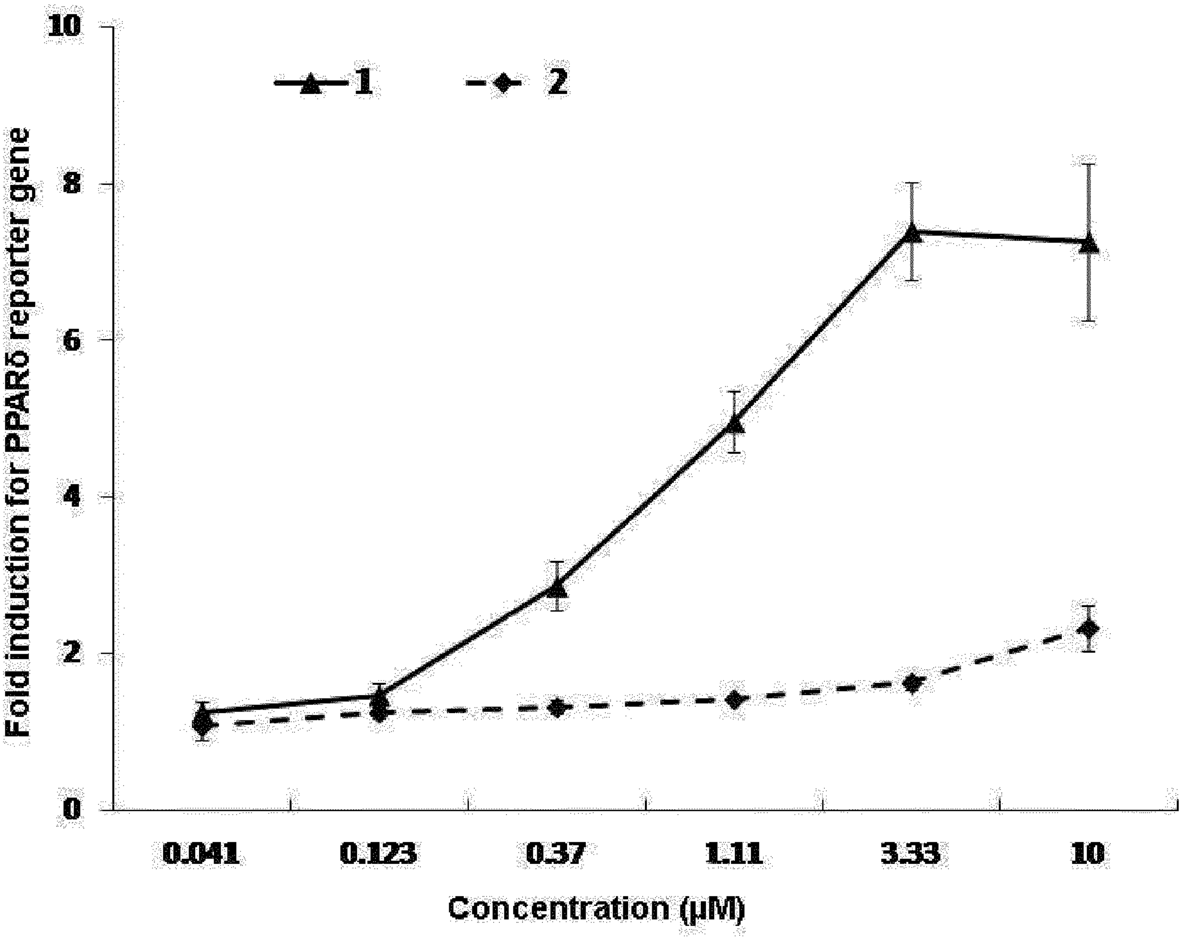
10
8
6
4
2
0
1
2
Fold induction for PPARδ reporter gene
0.041
0.123
0.37
1.11
3.33
10
Concentration (μM)

**Figure 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201610856914
- WO 0008002 A
- WO 0157001 A1
- US 6054453 A
- EP 088317 B1
- WO 9725042 A
- WO 0226729 A2
- US 6353018 B1
- CN 1257893 C
- CN 1257893


### Non-patent literature cited in the description


- **KELLER** ; **WAHLI**. *Trends Endocrin Metab*, 1993, vol. 4, 291-296
- **WANG YX et al.** *Cell*, 18 April 2003, vol. 113 (2), 159-70
- **LAN Y-K et al.** *Chinese J New Drugs*, 2004, vol. 13 (8), 718-720
- **MITSCHER LA et al.** *J Med Chem*, 1987, vol. 30 (12), 2283-6
- **CARTER RB et al.** *J Pharmacol Exp Ther*, 1985, vol. 234 (2), 299-306
- **TOBERT J. A. et al.** *Clin Pharmacol Ther*, 1981, vol. 29 (3), 344-50
- **PIETER A. et al.** *Drugs*, 1993, vol. 45 (4), 509-517
- **BLASCHKE G. et al.** *Arzneim-Forsch*, 1979, vol. 29 (10), 1640-2
- Remington's Pharmaceutical Sciences. Mack Publishing, 1995